# EUROPEAN PATENT SPECIFICATION

(11) **EP 3 634 296 B1**
(45) Date of publication and mention of the grant of the patent: **30.07.2025**
(21) Application number: 18814438.0
(22) Date of filing: 05.06.2018
(51) Int. Cl.: A61B 34/35, A61B 34/00, G10L 15/22, G16H 40/67, G16H 20/40, A61B 17/00, B25J 9/16, G10L 15/26, G16H 40/63

(54) **SYSTEMS AND METHODS FOR STATE-BASED SPEECH RECOGNITION IN A TELEOPERATIONAL SYSTEM**
SYSTEME UND VERFAHREN ZUR ZUSTANDSBASIERTEN SPRACHERKENNUNG IN EINEM TELEOPERATIONSSYSTEM
SYSTÈMES ET MÉTHODES DE RECONNAISSANCE VOCALE BASÉE SUR L'ÉTAT DANS UN SYSTÈME DE TÉLÉOPÉRATION

(30) Priority: 06.06.2017 US 201762515864 P
(43) Date of publication of application: 15.04.2020
(73) Proprietor: Intuitive Surgical Operations, Inc., Sunnyvale, CA 94086 (US)
(72) Inventor: ITKOWITZ, Brandon D., Sunnyvale, California 94086 (US); ARSANIOUS, Joseph M., Sunnyvale, California 94086 (US); BURNS, Christopher R., Sunnyvale, California 94086 (US)
(74) Representative: MacDougall, Alan John Shaw
(86) International application number: PCT/US2018/036146
(87) International publication number: WO 2018/226756

(56) References cited:
- WO-A1-2016/049294
- WO-A1-2017/083768
- WO-A2-2010/093152
- US-A- 5 544 654
- US-A- 5 970 457
- US-A1- 2005 114 140
- US-A1- 2006 142 740
- US-A1- 2009 036 902
- US-B1- 6 278 975

## Description

### CROSS-REFERENCE TO RELATED APPLICATIONS

This application claims the benefit of U.S. Provisional Application 62/515,864 filed June 6, 2017.

### FIELD

The present disclosure is directed to systems and methods for performing a teleoperational medical procedure and more particularly to systems and methods for providing state-based speech recognition during a teleoperational medical procedure.

### BACKGROUND

Minimally invasive medical techniques are intended to reduce the amount of tissue that is damaged during invasive medical procedures, thereby reducing patient recovery time, discomfort, and harmful side effects. Such minimally invasive techniques may be performed through natural orifices in a patient anatomy or through one or more surgical incisions. Through these natural orifices or incisions, clinicians may insert medical tools to reach a target tissue location. Minimally invasive medical tools include instruments such as therapeutic instruments, diagnostic instruments, and surgical instruments. Minimally invasive medical tools may also include imaging instruments such as endoscopic instruments. Imaging instruments provide a user with a field of view within the patient anatomy. Some minimally invasive medical tools and imaging instruments may be teleoperated or otherwise computer-assisted. During a teleoperated or computer-assisted procedure, a surgeon may require additional information, may need assistance with equipment or instrument, or may seek guidance in problem-solving. State-based speech recognition systems and methods, which evaluate the current context in which the surgeon in operating, may be used to provide the surgeon with accurate information in an efficient and safe manner.

### SUMMARY

The embodiments of the invention are summarized by the claims that follow the description.

According to the present invention, the teleoperational surgical system of claim 1 is provided. Further aspects of the present invention are set out in the dependent claims. It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTIONS OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion. In addition, the present disclosure may repeat reference numerals and/or letters in the various examples. This repetition is for the purpose of simplicity and clarity and does not in itself dictate a relationship between the various embodiments and/or configurations discussed.
FIG. 1A is a schematic view of a teleoperational medical system, in accordance with an embodiment of the present disclosure.
FIG. 1B is a perspective view of a patient side cart, according to one example of principles described herein.
FIG. 1C is a perspective view of a surgeon's control console for a teleoperational medical system, in accordance with many embodiments.
FIG. 2 illustrates a method for conducting a teleoperational medical procedure using state-based speech recognition.
FIG. 3 illustrates a method for using a teleoperational system to conduct a teleoperational procedure using state-based speech recognition.
FIG. 4 illustrates a method for using a teleoperational system to conduct a teleoperational procedure by initiating a speech recognition enabling signal.
FIG. 5 is a schematic view of a teleoperational medical system comprising multiple discrete subsystems responsive to and in communication with a speech recognition system.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. In the following detailed description of the aspects of the invention, numerous specific details are set forth in order to provide a thorough understanding of the disclosed embodiments. However, it will be obvious to one skilled in the art that the embodiments of this disclosure may be practiced without these specific details. In other instances well known methods, procedures, components, and circuits have not been described in detail so as not to unnecessarily obscure aspects of the embodiments of the invention.

Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. In addition, dimensions provided herein are for specific examples and it is contemplated that different sizes, dimensions, and/or ratios may be utilized to implement the concepts of the present disclosure. To avoid needless descriptive repetition, one or more components or actions described in accordance with one illustrative embodiment can be used or omitted as applicable from other illustrative embodiments. For the sake of brevity, the numerous iterations of these combinations will not be described separately. For simplicity, in some instances the same reference numbers are used throughout the drawings to refer to the same or like parts.

The embodiments below will describe various instruments and portions of instruments in terms of their state in three-dimensional space. As used herein, the term "position" refers to the location of an object or a portion of an object in a three-dimensional space (e.g., three degrees of translational freedom along Cartesian X, Y, Z coordinates). As used herein, the term "orientation" refers to the rotational placement of an object or a portion of an object (three degrees of rotational freedom - e.g., roll, pitch, and yaw). As used herein, the term "pose" refers to the position of an object or a portion of an object in at least one degree of translational freedom and to the orientation of that object or portion of the object in at least one degree of rotational freedom (up to six total degrees of freedom). As used herein, the term "shape" refers to a set of poses, positions, or orientations measured along an object.

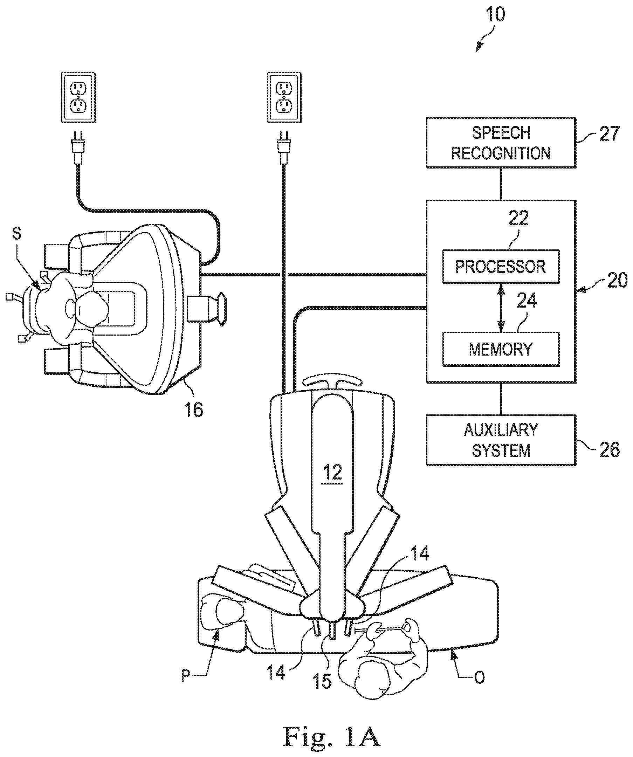

Referring to FIG.1A of the drawings, a teleoperational medical system for use in, for example, medical procedures including diagnostic, therapeutic, or surgical procedures, is generally indicated by the reference numeral 10. As will be described, the teleoperational medical systems of this disclosure are under the teleoperational control of a surgeon. In alternative embodiments, a teleoperational medical system may be under the partial control of a computer programmed to perform the procedure or sub-procedure. In still other alternative embodiments, a fully automated medical system, under the full control of a computer programmed to perform the procedure or sub-procedure, may be used to perform procedures or sub-procedures. As shown in FIG. 1A, the teleoperational medical system 10 generally includes a teleoperational assembly 12 mounted to or near an operating table O on which a patient P is positioned. The teleoperational assembly 12 may be referred to as a patient side cart. A medical instrument system 14 and an endoscopic imaging system 15 are operably coupled to the teleoperational assembly 12. An operator input system 16 allows a surgeon or other type of clinician S to view images of or representing the surgical site and to control the operation of the medical instrument system 14 and/or the endoscopic imaging system 15.

The operator input system 16 may be located at a surgeon's console, which is usually located in the same room as operating table O. It should be understood, however, that the surgeon S can be located in a different room or a completely different building from the patient P. In various embodiments, a teleoperational medical system may include more than one operator input system 16 and surgeon's console. In various embodiments, an operator input system may be available on a mobile communication device including a tablet or a laptop computer. Operator input system 16 generally includes one or more control device(s) for controlling the medical instrument system 14. The control device(s) may include one or more of any number of a variety of input devices, such as hand grips, joysticks, trackballs, data gloves, trigger-guns, foot pedals, hand-operated controllers, voice recognition devices, touch screens, body motion or presence sensors, and the like. In some embodiments, the control device(s) will be provided with the same degrees of freedom as the medical instruments of the teleoperational assembly to provide the surgeon with telepresence, the perception that the control device(s) are integral with the instruments so that the surgeon has a strong sense of directly controlling instruments as if present at the surgical site. In other embodiments, the control device(s) may have more or fewer degrees of freedom than the associated medical instruments and still provide the surgeon with telepresence. In some embodiments, the control device(s) are manual input devices which move with six degrees of freedom, and which may also include an actuatable handle for actuating instruments (for example, for closing grasping jaw end effectors, applying an electrical potential to an electrode, delivering a medicinal treatment, and the like).

The teleoperational assembly 12 supports and manipulates the medical instrument system 14 while the surgeon S views the surgical site through the console 16. An image of the surgical site can be obtained by the endoscopic imaging system 15, such as a stereoscopic endoscope, which can be manipulated by the teleoperational assembly 12 to orient the endoscope 15. A control system 20 can be used to process the images of the surgical site for subsequent display to the surgeon S through the surgeon's console 16. The number of medical instrument systems 14 used at one time will generally depend on the diagnostic or surgical procedure and the space constraints within the operating room among other factors. The teleoperational assembly 12 may include a kinematic structure of one or more non-servo controlled links (e.g., one or more links that may be manually positioned and locked in place, generally referred to as a set-up structure) and a teleoperational manipulator. The teleoperational assembly 12 includes a plurality of motors that drive inputs on the medical instrument system 14. These motors move in response to commands from the control system (e.g., control system 20). The motors include drive systems which when coupled to the medical instrument system 14 may advance the medical instrument into a naturally or surgically created anatomical orifice. Other motorized drive systems may move the distal end of the medical instrument in multiple degrees of freedom, which may include three degrees of linear motion (e.g., linear motion along the X, Y, Z Cartesian axes) and in three degrees of rotational motion (e.g., rotation about the X, Y, Z Cartesian axes). Additionally, the motors can be used to actuate an articulable end effector of the instrument for grasping tissue in the jaws of a biopsy device or the like. Instruments 14 may include end effectors having a single working member such as a scalpel, a blunt blade, an optical fiber, or an electrode. Other end effectors may include, for example, forceps, graspers, scissors, or clip appliers.

The teleoperational medical system 10 also includes a control system 20. The control system 20 includes at least one memory 24 and at least one processor 22, and typically a plurality of processors, for effecting control between the medical instrument system 14, the operator input system 16, and other auxiliary systems 26 which may include, for example, imaging systems, audio systems (including an intercom system), fluid delivery systems, display systems, mobile vision carts, illumination systems, steering control systems, irrigation systems, and/or suction systems. The control system 20 also includes programmed instructions (e.g., a computer-readable medium storing the instructions) to implement some or all of the methods described in accordance with aspects disclosed herein. While control system 20 is shown as a single block in the simplified schematic of FIG. 1A, the system may include two or more data processing circuits with one portion of the processing optionally being performed on or adjacent the teleoperational assembly 12, another portion of the processing being performed at the operator input system 16, and the like. Any of a wide variety of centralized or distributed data processing architectures may be employed. Similarly, the programmed instructions may be implemented as a number of separate programs or subroutines, or they may be integrated into a number of other aspects of the teleoperational systems described herein. In one embodiment, control system 20 supports wireless communication protocols such as Bluetooth, IrDA, HomeRF, IEEE 802.11, DECT, and Wireless Telemetry.

The control system 20 is in communication with or includes a speech recognition system 27. The speech recognition system 27 includes one or more microphones for receiving voice communications from personnel in the surgical environment, particularly the surgeon S. The speech recognition system may further include a one or more processors and one or more memory devices for processing the voice communications received by the microphone. Optionally, the processor 22 and memory 24 may process the voice communications received by the speech recognition system 27. The processors may include software and related hardware for receiving and interpreting voice communications from a surgeon and generating appropriate corresponding output signals. The microphones of the speech recognition system 27 may be located in in close proximity to the surgeon S or other surgical staff to reduce the amount of background noise provided to the processor. Optionally, one or more of the microphones may be mounted to a headset that is worn by the surgeon S or other surgical staff.

The speech recognition system 27 digitizes the oral voice communications received by the microphone, converting the voice communications into electronic form. The digitized words or sounds are analyzed and interpreted using natural language processing or other speech processing technologies. The analysis may include a comparison with a library of recognized words and sounds stored in in the memory of the speech recognition system or accessible to the speech recognition system over an internal network (e.g., a secured network of a medical facility or a teleoperational system provider) or an external network (e.g, the Internet).

In some embodiments, control system 20 may include one or more servo controllers that receive force and/or torque feedback from the medical instrument system 14. Responsive to the feedback, the servo controllers transmit signals to the operator input system 16. The servo controller(s) may also transmit signals instructing teleoperational assembly 12 to move the medical instrument system(s) 14 and/ or endoscopic imaging system 15 which extend into an internal surgical site within the patient body via openings in the body. Any suitable conventional or specialized servo controller may be used. A servo controller may be separate from, or integrated with, teleoperational assembly 12. In some embodiments, the servo controller and teleoperational assembly are provided as part of a teleoperational arm cart positioned adjacent to the patient's body.

The control system 20 can be coupled with the endoscope 15 and can include a processor to process captured images for subsequent display, such as to a surgeon on the surgeon's console, or on another suitable display located locally and/or remotely. For example, where a stereoscopic endoscope is used, the control system 20 can process the captured images to present the surgeon with coordinated stereo images of the surgical site. Such coordination can include alignment between the opposing images and can include adjusting the stereo working distance of the stereoscopic endoscope.

In alternative embodiments, the teleoperational system may include more than one teleoperational assembly and/or more than one operator input system. The exact number of manipulator assemblies will depend on the surgical procedure and the space constraints within the operating room, among other factors. The operator input systems may be collocated, or they may be positioned in separate locations. Multiple operator input systems allow more than one operator to control one or more manipulator assemblies in various combinations.

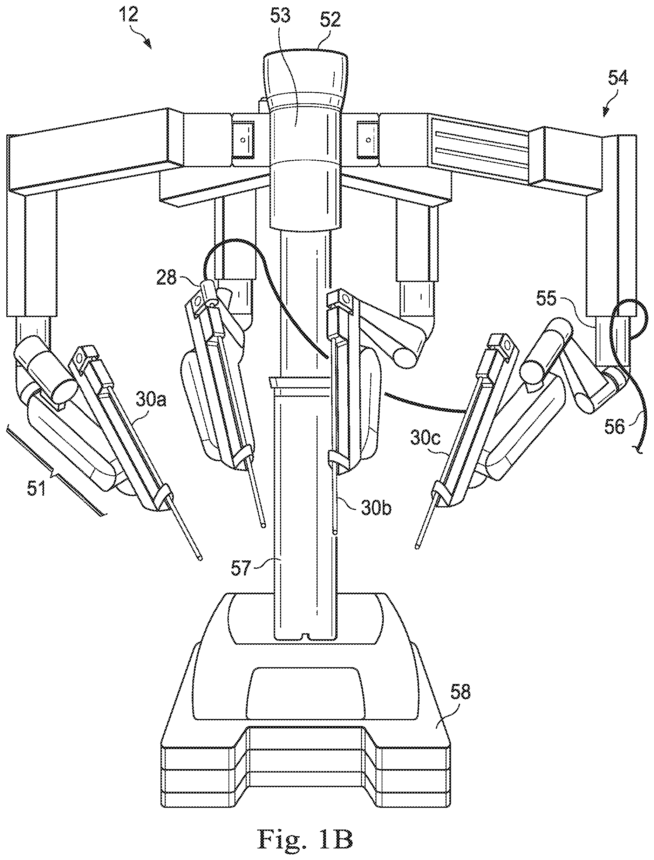

FIG. 1B is a perspective view of one embodiment of a teleoperational assembly 12 which may be referred to as a patient side cart. The patient side cart 12 shown provides for the manipulation of three surgical tools 30a, 30b, 30c (e.g., instrument systems 14) and an imaging device 28 (e.g., endoscopic imaging system 15), such as a stereoscopic endoscope used for the capture of images of the site of the procedure. The imaging device may transmit signals over a cable 56 to the control system 20. Manipulation is provided by teleoperative mechanisms having a number of joints. The imaging device 28 and the surgical tools 30a-c can be positioned and manipulated through incisions in the patient so that a kinematic remote center is maintained at the incision to minimize the size of the incision. Images of the surgical site can include images of the distal ends of the surgical tools 30a-c when they are positioned within the field-of-view of the imaging device 28.

The patient side cart 12 includes a drivable base 58. The drivable base 58 is connected to a telescoping column 57, which allows for adjustment of the height of the arms 54. The arms 54 may include a rotating joint 55 that both rotates and moves up and down. Each of the arms 54 may be connected to an orienting platform 53. The orienting platform 53 may be capable of 360 degrees of rotation. The patient side cart 12 may also include a telescoping horizontal cantilever 52 for moving the orienting platform 53 in a horizontal direction.

In the present example, each of the arms 54 connects to a manipulator arm 51. The manipulator arms 51 may connect directly to a medical instrument 30a. The manipulator arms 51 may be teleoperatable. In some examples, the arms 54 connecting to the orienting platform are not teleoperatable. Rather, such arms 54 are positioned as desired before the surgeon 18 begins operation with the teleoperative components.

Endoscopic imaging systems (e.g., systems 15, 28) may be provided in a variety of configurations including rigid or flexible endoscopes. Rigid endoscopes include a rigid tube housing a relay lens system for transmitting an image from a distal end to a proximal end of the endoscope. Flexible endoscopes transmit images using one or more flexible optical fibers. Digital image based endoscopes have a "chip on the tip" design in which a distal digital sensor such as a one or more charge-coupled device (CCD) or a complementary metal oxide semiconductor (CMOS) device store image data. Endoscopic imaging systems may provide two- or three- dimensional images to the viewer. Two-dimensional images may provide limited depth perception. Three-dimensional stereo endoscopic images may provide the viewer with more accurate depth perception. Stereo endoscopic instruments employ stereo cameras to capture stereo images of the patient anatomy. An endoscopic instrument may be a fully sterilizable assembly with the endoscope cable, handle and shaft all rigidly coupled and hermetically sealed.

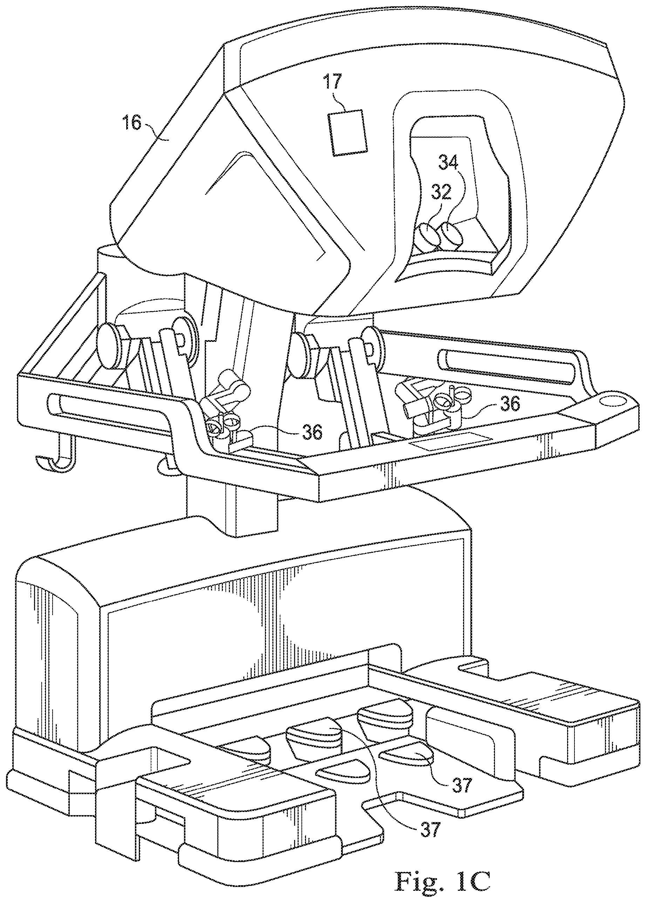

FIG. 1C is a perspective view of the surgeon's console 16. The surgeon's console 16 includes a left eye display 32 and a right eye display 34 for presenting the surgeon S with a coordinated stereo view of the surgical environment that enables depth perception. The console 16 further includes one or more input control devices 36, which in turn cause the teleoperational assembly 12 to manipulate one or more instruments or the endoscopic imaging system. The input control devices 36 can provide the same degrees of freedom as their associated instruments 14 to provide the surgeon S with telepresence, or the perception that the input control devices 36 are integral with the instruments 14 so that the surgeon has a strong sense of directly controlling the instruments 14. To this end, position, force, and tactile feedback sensors (not shown) may be employed to transmit position, force, and tactile sensations from the instruments 14 back to the surgeon's hands through the input control devices 36. Input control devices 37 are foot pedals that receive input from a user's foot.

During a teleoperational procedure, a surgeon may require additional information, may need assistance with equipment or instrument, or may seek guidance in problem-solving. Current trouble-shooting or information gathering techniques require a surgeon to suspend the surgical activity to seek information or resolve problems. For example, if the surgeon is encountering limitations or resistance in the medical instrument while engaged with the operator console 16, the surgeon may need to interrupt the surgical procedure, move away from the operator console, release the control devices 36 to access on-line troubleshooting menus or manuals, or otherwise delay the procedure and introduce associated risk. As described in detail below, a speech recognition system that is aware of the current status of the procedure and of the teleoperational system components may allow the surgeon to access information and troubleshoot problems more efficiently and safely.

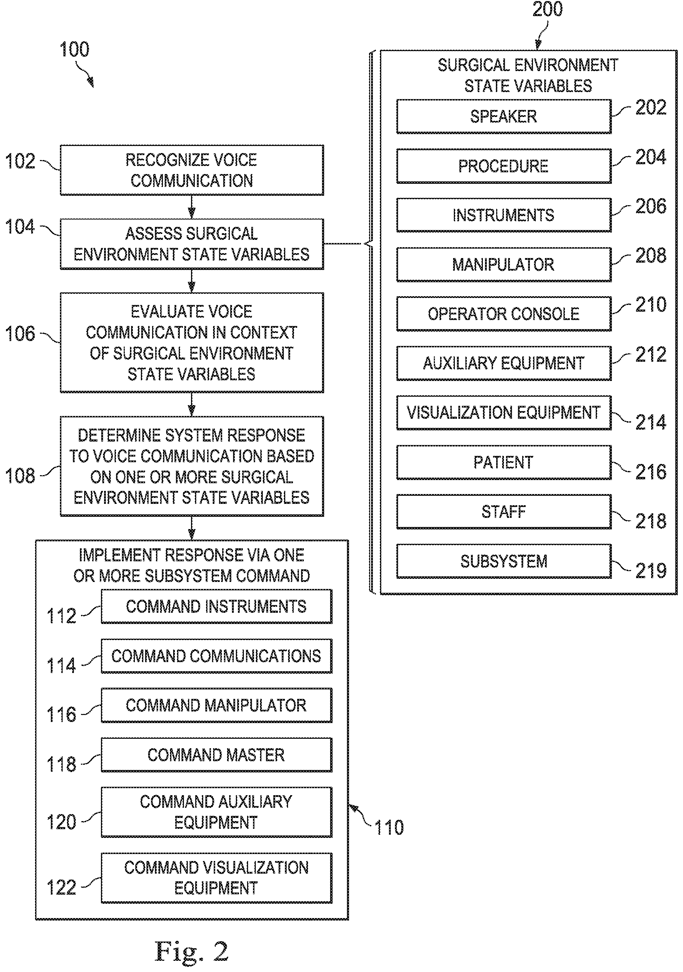

FIG. 2 illustrates a method 100 for using the teleoperational system 10 to conduct a teleoperational procedure using state-based speech recognition. The method 100 is illustrated in FIG. 2 as a set of operations or processes. Not all of the illustrated processes may be performed in all embodiments of method 100. Additionally, one or more processes that are not expressly illustrated in FIG. 2 may be included before, after, in between, or as part of the illustrated processes. In some embodiments, one or more of the processes of method 100 may be implemented, at least in part, in the form of executable code stored on non-transitory, tangible, machine-readable media that when run by one or more processors (e.g., the processors of control system 20) may cause the one or more processors to perform one or more of the processes.

At a process 102, a voice communication in the surgical environment is recognized by the control system (e.g., control system 20). More specifically, the speech recognition system 27 may detect voice communication from the surgeon S or another member of the surgical team. The detected voice communication is analyzed and interpreted by the speech recognition system 27 and/or the control system 20. U.S. Patent No. 6,591,239 (filed December 9, 1999) (disclosing "Voice Controlled Surgical Suite") discloses one such speech recognition system.

At a process 104, a variety of surgical environment state variables 200 may be monitored and assessed by the control system 20. The variables 200 provide information about the state of various systems, instruments, equipment, procedures, and people within the surgical environment. A speaker state variable 202 provides information about the speaker of the voice communication. The speaker may be anyone on the surgical team including the surgeon S and/or the surgical staff. The information about the speaker may include identification information, training history, credentials, procedure history, typical surgical team members, communication preferences, frequently used vernacular/jargon, anthropometric information, ergonomic preferences, equipment preferences, interface preferences, and the speaker's physical location in the surgical environment, including proximity to systems and instruments. The training history may include, for example, a cumulative record of the user's simulator experience and proctor-assisted procedure experience, including the types of procedures, the outcome of the procedures, and any issues occurring during the procedures. It may also include evaluations, certifications, and a cumulative log of hours in training. The training history may be updated after each training episode for a user. The credential information may include, for example, credentials or other rights to use the systems or to access specific procedures with those systems. Credentials may be issued by an issuing authority such as a trainer, a medical facility (e.g., a hospital, clinic, training center). The procedure history information may include, for example, a cumulative record of the procedures performed by the speaker including types of procedures, any user idiosyncrasies, procedure outcomes, and previously recognized voice communications. The procedure information may include a count of procedures performed, types of procedures performed, speed of procedures performed, and transition times for prior procedures. The procedure information may further include the software version and model of the system used for each prior procedure. The communication preferences may include a record of the languages in which the speaker is fluent and preferred languages for audio and/or textual communication. The communication preferences may also include the speaker's preferences regarding the medium for delivery of communication (e.g., visual, auditory, combined visual and auditory) and volume settings. The anthropometric information may include anatomic measurement information for the speaker including, for example, optometric measurements of vision and any needed corrective lenses, intraocular spacing, height, weight, handedness, and physical limitations including hearing or vision. The ergonomic preferences may include operator control and instrument settings that the speaker finds to be most comfortable or useful. The equipment preferences information may include the speaker's preferences regarding optional arrangements, functions, and settings of components of a teleoperational system (e.g., system 10, instrument system 14, user console 16). For example, the equipment preferences may include preferred hand positions and button/pedal function assignments for the control console 16. Preferences may include the speaker's preferred configuration of the assembly 12 relative to the patient. Preferences may include preferred instrument (e.g., instrument 14) settings such as ablation power levels, energy, force, torque, staple cartridge, and handedness for stapler. The preferences may include preferred port placements and arm configurations. The preferences may include preferred functionality such as preferred table angles , patient positioning presets, or microsurgery capability. The preferences may include preferred auxiliary equipment (e.g., equipment 26) including supplemental imaging systems (e.g., MRI, x-ray, ultrasound); video input and output; insufflation settings (e.g., desire pressure, maximum flow rate), audio settings (e.g., which microphones activated, feedback suppression, which speakers activated, use of voice prompts). The user interface preferences may include the speaker's preferences regarding the graphical user interface, other sensory displays, or the endoscopic instrument settings. For example, preferences may relate to vision correction and autofocus. Preferences may also include the speaker's preferred display color, brightness, contrast, shadow, dynamic contrast, and use of near infrared imaging.

The speaker state variable 202 may also include information about the intelligibility of the speaker's speech. Speech intelligibility may be influenced by speech characteristics such as dialect, accent, or speech impediment or may be influenced by physical impediments such as a surgical mask over the speaker's face or microphone distortion. For example, a speaker state variable may include whether the speaker has a speech impediment like rhotacism (e.g., chronic mispronunciation of specific consonant like "r") that impacts word pronunciation is a predictable way. In another example, a speaker state variable may include whether speaker is wearing a surgical mask, has a preference for wearing a surgical mask, and/or has a predictable change in speech intelligibility when wearing a surgical mask.

A procedure state variable 204 provides information about the surgical procedure including, for example, information about the planned sequence of tasks performed in the procedure, common technique variations for conducting the procedure, common issues that arise during the procedure, and tool changes needed during the procedure. The procedure state variable 204 may also provide information to track devices used in the procedure. For example, the procedure state variable may include information regarding the location of clamps, sutures, other surgical devices deposited within the patient anatomy during the surgical procedure.

The instrument state variable 206 includes information about the instrument (e.g., instrument 14) or instruments for past, current, or future use in the surgical procedure. The information may include instrument identification information, configurations, operational settings, and common failure modes. The instrument state variable 206 may include information about alternative names used to identify instruments, the instrument range of motion, and kinematic information such as the current location of the instrument tips. The instrument state variable 206 may include information about what an instrument is currently doing and whether a command associated with a voice communication is feasible or would cause damage to the patient or another portion of the surgical system.

The manipulator state variable 208 includes information about the teleoperational manipulator (e.g., manipulator 12) including, for example, the configuration of each arm, the movement range of each arm, the instrument attached to each manipulator arm, and common failure modes for the manipulator. The variable 208 may also include information about the range of motion of a manipulator arm and whether motion is obstructed by another object in the surgical environment.

The operator console state variable 210 includes information about the operator input system (e.g., system 16) including, for example, information about the functional assignment of the control devices 36, 37, the degrees of freedom of movement associated with each control device, the images visible through the eye displays 32, 34, the range of movement for each control device, and common failure modes for the control devices or other aspects of the operator input system. The variable 210 may further include information about the volume or mute status of any speakers in the operator input system, whether a dual operator in system is in use and which station is currently in control,

The auxiliary equipment state variable 212 includes information about the auxiliary equipment (e.g., systems 26) which may include configuration, setting, power, and failure mode information about imaging systems, audio systems, fluid delivery systems, display systems, illumination systems, steering control systems, irrigation systems, and/or suction systems in use in the surgical environment.

The visualization equipment state variable 214 includes information about the endoscopic imaging system (e.g., system 15) and any associated display systems. The information may include, for example, pose information about the distal end of the endoscope in the patient anatomy, illumination settings, image processor settings, heat discharge information, power status, optical configuration, and common failure modes.

The patient state variable 216 includes information about the current patient including, for example, identification, height, weight, body mass index, gender, surgical history, medical history, location of current surgical ports, and pose of patient relative to the manipulator.

The staff state variable 218 includes information about the staff in the surgical environment including identification information, assigned tasks, assigned inventory, physical location within the surgical environment, training history, credentials, procedure history, communication preferences, anthropometric information, ergonomic preferences, equipment preferences, and interface preferences.

The subsystem variable 219 includes information about subsystems in the surgical environment. The subsystem may include, for example, the surgeon console 16, an auxiliary surgeon console, a teleoperational assembly 12, a vision cart, or a mobile computing device. Each subsystem includes its own controllable devices including displays, speakers, microphones, instruments, and/or power supplies. Identifying the subsystem allows the voice communication to be interpreted in a subsystem dependent manner. Each subsystem may be associated with its own command set such that only voice communications that include commands within the associated command set may elicit a response from the subsystem. For example, if the voice command refers colloquially to "Arm 1," the system state variables may be assessed to determine which subsystem is associated with that identifier and a system response may be directed to the determined subsystem. If the voice command is "Swap needle driver," the system state variable may be assessed to determine which subsystem includes a needle driver, and a system response may be directed to the determined subsystem.

At a process 106, the voice communication is evaluated in the context of the surgical environment state variables 200. More specifically, one or more of the variables 200 are used, for example, to determine the meaning of the voice communication, answer a question posed by the voice communication, trouble-shoot a problem identified in the voice communication, execute a command made in the voice communication, resolve ambiguity raised in the voice communication, identify warnings associated with the voice communication, and/or provide auditory or textual instructions to another team member in the surgical environment. The meaning of the voice communication may be determined by reference to word recognition search space or library. Words in the word recognition search space may be promoted or prioritized for matching with the voice communication based on the assessed surgical environment state variables. Words in the word recognition search space are associated with output commands to the various components of the surgical system. The word recognition search space may be constrained by the surgical state variables so that system responses not associated with the variable constraints may be eliminated from consideration when determining a response.

Evaluating the voice communication in the context of the surgical environment state variables may include limiting a word recognition search space based upon the variables. For example, if the assessment of the instrument surgical state variable indicates that the instruments in the surgical space are graspers and cautery shears only, the term "sealer" may be eliminated from word recognition search space to avoid confusion between the terms "shears" and "sealer." As another example, if the assessment of the instrument surgical state variable indicates that a monopolar curved scissors is in use, alternative names and known jargon such as "MCS," "scissors," "shears," "hot shears," and "cautery shears" are prioritized as potential matches with the recognized voice communication.

Evaluating the voice communication in the context of the surgical environment state variables may also include evaluating parts of speech including nouns, verbs, and demonstrative pronouns such as "this" and "that." For example, if the surgeon asks, "What is wrong with this?" while gesticulating with the right hand user control device, the term "this" may be evaluated in the context of the manipulator state variable for the manipulator arm associated with the right hand user control device, the instrument state variable for the instrument attached to the manipulator arm associated with the right hand user control device, and the master console state variable for the right hand user control to troubleshoot potential issues in the chain of control of the instrument controlled by the right hand user control device. As another example, if the surgeon asks, "What is wrong with that?" while gesticulating with the right hand user control device to point to the instrument controlled by the left hand user control device, the term "that" may be evaluated in the context of the manipulator state variable for the manipulator arm associated with the left hand user, the instrument state variable for the instrument attached to the manipulator arm associated with the left hand user control device, and the master console state variable for the left hand user control to troubleshoot potential issues in the chain of control of the instrument controlled by the left hand user control device.

Evaluating the voice communication in the context of the surgical environment state variables may also include evaluating internally directed instructions (e.g., "da Vinci, make screen brighter") and externally directed instructions (e.g., "Nurse, reload stapler.") The internal or external nature of the instructions may be identified by leading key words such as "da Vinci" (indicating a command to the teleoperational control system) or "Nurse" (indicating a command to a surgical staff member). Alternatively, leading key words may be omitted and the internal or external nature of the instructions may be determined by review of the surgical variables such as variables 218, 204, 206 to determine which commands require system or human action.

Evalutating the voice communication in the context of the surgical environmental state variables may also include evaluating the voice communication in the context of speech intelligibility factors. Speech recognition algorithms may be developed to recognize and/or correct for errors due to speech intelligibility. For example, when evaluating the voice communication, the system may select between multiple speech recognition models based upon whether the speaker is wearing or customarily wears a mask. The speech recognition model for mask wearers may compensate for the effects of a muffled voice or the dropping of consonants at the beginning of some words. In various embodiments, the system may evaluate the speech with both speech recognition models and adaptively select the model that generates more accurate speech recognition. Accuracy may be based on surgical context. For example, ambiguity between "arm" and "farm" may be resolved as "arm" due to the surgical context. Accuracy may also be based on procedural context. For example, "reposition the patient" may be a more appropriate interpretation than "reposition the station" based on the state of the surgical procedure. Accuracy may also be based on grammar or meaning. For example, "introduce the pouch" may be recognized as grammatically preferable to "introduce the ouch."

At a process 108, the system response to the recognized voice communication is determined based on one or more of the surgical environment state variables. The appropriate system response may be determined to be, for example, a command to control the motion of an instrument, to control motion of a manipulator arm, to control operation of auxiliary equipment, to make an adjustment to the endoscope, to send a textual or voice communication to a surgical staff member or another user, to update a patient record, to provide one or more follow-up inquiries to the speaker (e.g., via voice or text communication) to resolve ambiguity in or clarify/confirm the original voice communication. Determining the system response may include developing and presenting choices of system response to the speaker in order based on a confidence factor associated with a plurality of candidate responses.

At a process 110, the determined system response is implemented with one or more commands to one or more subsystems of the surgical system. For example, the determined system response may be implemented via a command 112 to control an instrument, a command 114 to control a textual or auditory communication to a user (including a user not present in the surgical area), a command 116 to control a manipulator arm, a command 118 to control a user control device, a command 120 to control the operation of auxiliary equipment, and/or a command 122 to control visualization equipment including the endoscope.

Multiple examples of the method 100 are provided below.

In one example, if the voice communication, "too dark" is received, surgical state variables associated with the visualization equipment 214 are assessed and evaluated. Options for system response associated with recognized voice communication may include increasing the illumination of the endoscope or adjusting the digital image processor to increase brightness. Because the variable 214 also includes information about the pose of the distal end of the illuminator, the appropriate response may be determined by the distance between the distal end of the illuminator and the patient tissue. If the distance is greater than a predetermined threshold, implementing a command to increase the brightness of the illuminator may be appropriate, but if the distance is less than the predetermined threshold increasing the brightness of the illuminator may generate heat that will dry or burn the patient tissue. In such a case, adjusting the digital image processor to increase the brightness of the image displayed to the speaker may be more appropriate.

In another example, if the voice communication, "can't hear my assistant" is received, the verb "hear" is associated with a various auditory related variables including the speaker 202, the master console 210, the auxiliary equipment 212, and the staff 218. For example, surgical state variables associated with surgical staff 218 may be evaluated to determine which of one or more speaking surgical staff members is indicated. Surgical state variables associated with the speaker may be evaluated to determine whether the speaker has a known hearing deficiency. Surgical state variables associated with the master console 210 may be evaluated to determine whether the volume setting on speakers used by the surgeon can be adjusted. Surgical state variables associated with the auxiliary equipment 212 may be evaluated to determine whether a staff member's microphone is muted or may be adjusted.

In another example, if the voice communication, "change shears on arm one" is received, variables associated with "change," "shears," and "on arm one" are evaluated in the context of multiple surgical state variables including the procedure 204, and instruments 206, manipulator 208. For example, surgical state variables 206 and 208 may be evaluated to determine whether a shearing instrument is coupled to arm one or whether the speaker has made a mistake in associating the instrument with the manipulator arm. If there is no mistake, the implemented response may be to command an ejection of the shears. If there appears to be a mistake, the implemented response may be to highlight the correct arm with the coupled shears on a display to the speaker and query the speaker to confirm whether the highlighted arm is the appropriate arm on which to implement tool ejection. If evaluation of the procedure state variable 204 or the instrument state variable 206 indicates that the instrument is currently grasping patient tissue, the implemented response may be a refusal to execute the speakers command due to patient safety concerns or may be a command to the instrument to release the grasped tissue before commanding the manipulator arm to eject the instrument. A similar evaluation may occur if the voice communication orders the movement of the patient table. If the evaluated state variables indicate that an instrument is currently grasping tissue, the command to move the patient table may be refused or a tissue release command may first be implemented.

In another example, if the voice communication, "the instrument won't move correctly" is received, variables associated with "instrument" and "won't move correctly" are evaluated in the context of multiple surgical state variables including the procedure 204, instruments 206, manipulator 208, visualization equipment 214, and master console 210. For example, an evaluation of the manipulator variables 208 may indicate that two manipulator arms have come into contact or that movement of one of the manipulator arms is impinged upon by another piece of equipment in the surgical environment or a range of motion limitation. Alternatively, an evaluation of the master console variables 210 may indicate that the operator control devices are attempting to move outside of a permitted range of motion or that another operator in a dual console system currently has control. Alternatively, an evaluation of the instrument variables 206 may indicate that the instrument is not properly engaged with the manipulator arm or that the attempted movement is outside the instrument range of motion. Alternatively, an evaluation of the procedure variables 204, manipulator variables 208, and/or the visualization equipment variables 214 may indicate that the endoscope manipulator arm is activated (e.g. clutched), thus deactivating the other instrument arms. If the evaluation of variables determines that the manipulator arms are contacting each other, the determined and implemented system response may be to clutch the manipulator arm and readjust, change to a different endoscopic viewing angle, adjust or create a new access port, swap instruments between manipulator arms, or swap manipulator arms between ports.

In another example, if the voice communication, "the ESU isn't working" is received, the acronym ESU may be recognized as referring to an electrosurgical unit (e.g. a type of auxiliary equipment). The variables 212, 210 associated with the electrosurgical unit may be evaluated to determine whether there is electrical power being provided to the unit; whether the power is set insufficiently high for the commanded procedure; whether the foot pedal control on the operator console is malfunctioning whether the effect level has not been set and therefore defaulted to zero; whether the energy cable between the instrument and ESU is connected; or whether the energy pedal is actuated while the operator's head is not detected at the console viewer.

In another example, if the voice communication, "correct color" is received, variables associated with color including patient 214 and visualization equipment 214 may be evaluated. For example, an evaluation of the patient variable 214 may indicate that the patient is obese which allows the system to recognize that fat tissue is present which often appears with an orange colored hue. The determined system response may be to digitally adjust the color settings on the image processor.

In another example, if the voice communication is a set of instructions for one or more members of the surgical staff, variables associated with the staff 218 and the procedure 204 may be evaluated to determine to whom the instructions are addressed, the location of the staff member to whom the instructions are addressed, and where the instructions should be stored or displayed. The determined and implemented system response may be to generate an instruction log that is electronically sent to or accessible by one or more members of the surgical staff. If the member of the surgical staff is equipped with a mobile device (e.g., cell phone, tablet device), the presence of that mobile device in the surgical environment may be tracked and if it is not detected (e.g., the surgical staff member has left the room), the instructions may be transmitted to voice mail or transcribed as a text message and sent to the mobile device.

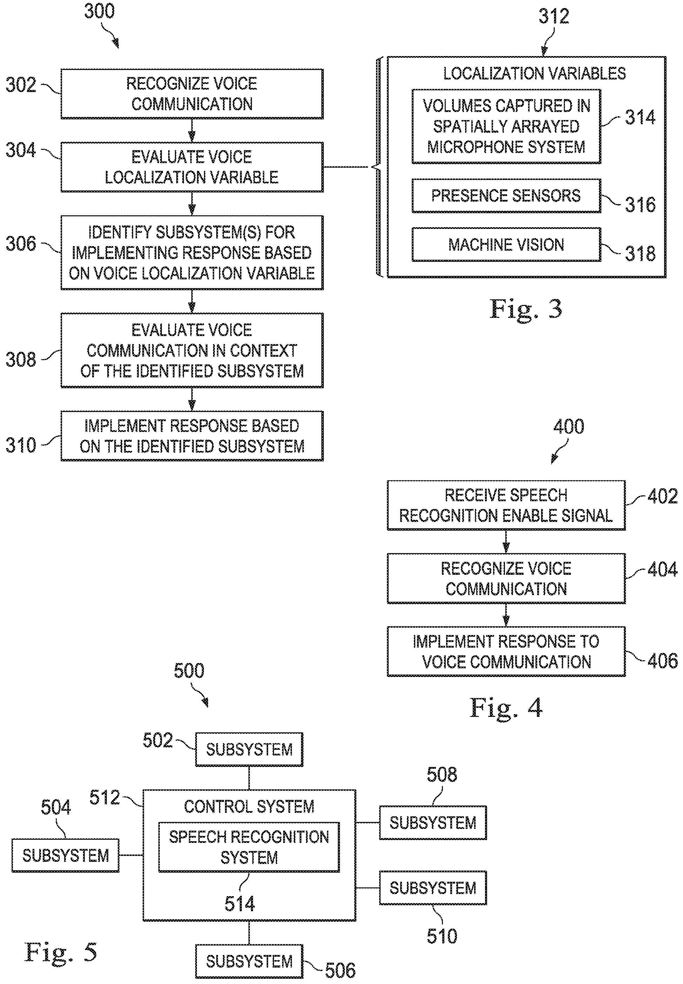

FIG. 3 illustrates a method 300 for using the teleoperational system 10 to conduct a teleoperational procedure using state-based speech recognition, particularly a voice localization variable. The method 300 is illustrated in FIG. 3 as a set of operations or processes. Not all of the illustrated processes may be performed in all embodiments of method 300. Additionally, one or more processes that are not expressly illustrated in FIG. 3 may be included before, after, in between, or as part of the illustrated processes. In some embodiments, one or more of the processes of method 300 may be implemented, at least in part, in the form of executable code stored on non-transitory, tangible, machine-readable media that when run by one or more processors (e.g., the processors of control system 20) may cause the one or more processors to perform one or more of the processes.

At a process 302, a voice communication in the surgical environment is recognized by the control system (e.g., control system 20). More specifically, the speech recognition system 27 may detect voice communication from the surgeon S or another member of the surgical team. The detected voice communication is analyzed and interpreted by the speech recognition system 27 and/or the control system 20.

At a process 304, a voice localization variable 312 is evaluated. The voice localization variable may be, for example, a speaker state variable 202. A voice localization variable may be any information that provides an indication of the speaker's location within the surgical environment of the system 10 or relative to equipment or instruments in the surgical environment. For example, a localization variable 314 is a set of audio volumes captured by a spatially separated microphone array. The speaker's location relative to the known positions of the microphones in the array may be determined by comparing the audio volume detected by each microphone in the array at a given time. For example, a louder sound detected by one of the microphones in the array may indicate that the speaker is closer to that microphone than another microphone at which the quieter sound is detected at the same time. A time delay measurement may also indicate proximity and therefore may be used as a voice localization variable. Additionally or alternatively, a localization variable 316 is a presence sensor associated with equipment in the system 10. For example the presence sensor may be a head-in presence sensor that detects that a user's head is in place for operating the surgeon's console 16. Additionally or alternatively, a localization variable 318 is machine vision information. For example, a machine vision system may include a camera system that observes the field near each microphone. The camera and microphone are assumed to have similar geometry for acquisition such that the microphone does not pick up sound that is substantially outside the field of view of the associated camera. The camera system continuously acquires and processes images to match features apparent in the image against a generic template of a face or facial features to determine if there is a high likelihood of a person in the image. Machine vision can also be used to identify specific individuals in the image associated with each microphone by comparing against a set of representative facial images of each person. In other embodiments, localization variables may be determined from sensors or identifiers coupled to the speaker such as radio frequency identification tags, optical sensors, or electro-magnetic position sensors.

At a process 306, a subsystem of the system 10 for providing the response to the voice communication is identified from the voice localization variable. The subsystem may include, for example, the surgeon console 16, an auxiliary surgeon console, a teleoperational assembly 12, a vision cart, or a mobile computing device. Each subsystem includes its own controllable devices including displays, speakers, microphones, power supplies. Identifying the subsystem allows the voice communication to be interpreted in a subsystem dependent manner. Each subsystem may be associated with its own command set such that only voice communications that include commands within the associated subset may elicit a response from the subsystem.

At a process 308, the voice communication is evaluated in the context of the identified system. For example, if an evaluation of the voice localization variable indicates that the speaker is located patient side rather than at the surgeon console, a voice communication requesting a surgical image may cause the image to be displayed on a patient side vision cart rather than on a display at the surgeon console. Subsequent voice communications to control display brightness or a zoom function would be applied to the image on the patient side cart rather than other displays not visible to the speaker. In another example, the voice communication may be used to transfer control of the identified system. For example, a voice communication such as "Take control of Arm 1" or "Take control of all arms" may be evaluated as a command to transfer control authority to the console subsystem where the speech is detected. Similarly in a dual operator console configuration, the voice communication, "Give control to the other console" or "Give control to Dr. Jones" may be evaluated as a command to transfer control from the console subsystem where the speech is detected to the second console subsystem or to the console subsystem into which Dr. Jones is logged.

Evaluating the voice communication in the context of the context of the identified subsystem may also include evaluating demonstrative pronouns such as "this" and "that." For example, if the surgeon asks, "What is wrong with this?" while physically located near a display system, the term "this" may be evaluated in the context of speaker's location in addition to recent activity of the display system and the settings of the display system. Thus the system may troubleshoot potential issues related to the display system such as powered state, brightness, displayed image, etc.

At a process 310, the response to the voice communication is implemented based on the voice localization variable via the identified subsystem. Subsystem dependent responses may be limited to command sets associated with the subsystem. For example, subsystem dependent responses may include commands to authorize control of the subsystem or instruments attached to the subsystem; to change a setting (e.g., display brightness, audio volume); to mute/un-mute an intercom microphone; to show or hide status messages; to set a subsystem value (e.g., an illumination level or an insufflation pressure); retrieve a value (e.g., an insufflation pressure or a temperature); adjust a value (e.g., a display brightness or a speaker volume); initiate a configuration (e.g., a set-up configuration); set a display mode (e.g., tile display, fluorescent image, uni-ocular, stereoscopic); retrieve a status (e.g. a clock time, an elapsed time, a recording on/off status); to troubleshoot (e.g., "why can't I move this instrument"); to acknowledge system messages (e.g. confirm content of warning message); to perform an instrument exchange (e.g., eject a tool, release a grip, swap an arm); to perform another discrete action (e.g., flip the endoscope angle, level a view, take images, annotate images, start/stop recordings, adjust zoom, adjust camera position, adjust master/slave scaling; or to perform advanced controls (e.g., table motion, optimize manipulator position, initiate ergonomic settings). Implementing the response may also include disabling components associated with non-identified subsystems.

FIG. 4 illustrates a method 400 for using the teleoperational system 10 to conduct a teleoperational procedure by initiating a speech recognition enabling signal. The method 400 is illustrated in FIG. 4 as a set of operations or processes. Not all of the illustrated processes may be performed in all embodiments of method 400. Additionally, one or more processes that are not expressly illustrated in FIG. 3 may be included before, after, in between, or as part of the illustrated processes. In some embodiments, one or more of the processes of method 400 may be implemented, at least in part, in the form of executable code stored on non-transitory, tangible, machine-readable media that when run by one or more processors (e.g., the processors of control system 20) may cause the one or more processors to perform one or more of the processes.

At a process 402, a speech recognition system enablement signal is received. The speech recognition system enablement signal may be, for example, a spoken trigger word or engagement of a physical trigger. For example the existing master clutch finger switch may be engaged to enable the speech recognition system. The master clutch finger switch may be located on one of the input control devices 36. The typical function of the master clutch finger switch is to interrupt the control loop linking the master control movement with the slave movement. This interruption allows the control devices to be repositioned. In this context, activation of the master clutch finger switch may have a secondary effect, namely that of enabling the speech recognition system. Optionally, following the activation of the master clutch finger switch, an uncharacteristic action or lack of action may further be required to enable the speech recognition system. For example, characteristically, activation of the master clutch finger switch is followed by repositioning of the control devices 36. If a predetermined period of time elapses without movement of the control devices 36, the activation of the master clutch finger switch may be recognized as a signal to enable the speech recognition system. Alternatively, the speech recognition system may be activated upon actuation of the master clutch but suspend activation (i.e., ignore speech) when the control system observes displacement of the control device beyond a threshold displacement value. Ignoring speech communication during active master clutch motion prevents acting upon erroneous or unintentional speech while also avoiding generating error feedback if partial or unrecognized speech detected. In other embodiments, an audible tone may be provided to alert the user that the speech recognition system is enabled and listening. In some embodiments, the master clutch finger switch is held is an activated state while the speech recognition system is enabled and listening.

At a process 404, a voice communication in the surgical environment is recognized by the control system (e.g., control system 20). More specifically, the speech recognition system 27 may detect voice communication from the surgeon S or another member of the surgical team. The detected voice communication is analyzed and interpreted by the speech recognition system 27 and/or the control system 20.

At a process 406, the response to the voice communication is implemented. In various embodiments, implementing the response may include suppressing other components of the system 10. For example, when the master clutch finger switch is activated at the surgeon console 16, the surgical suite intercom system may be suppressed so that spoken voice communication intended to provide system voice control is not broadcast to personnel in the surgical environment. This suppression avoids confusing the surgical personnel and reduces the risk that the surgical personnel will hear the commands and attempt to take corresponding action. In another alternative, a dedicated switch may activate a menu system that enables the detection of voice communication.

FIG. 5 illustrates a schematic view of a teleoperational medical system 500 comprising multiple discrete subsystems 502, 504, 506, 508, 510 responsive to and in communication with a control system 512 (e.g., system 20) that includes or is in communication with a speech recognition system 514 (e.g., system 27). The subsystems 502, 504, 506 may be, for example, teleoperational assemblies substantially similar to a teleoperational assembly 12 and may include one or a plurality of teleoperational arms. The subsystems 508, 510 may be, for example, operator input systems substantially similar to input system 16. Additional or alternative subsystems may include a display system, a mobile computing device, or an auxiliary system (e.g., system 26). An operator of the system 500 may issue voice commands recognized by the speech recognition system 514 (as previously described for system 27). Based on the recognized voice commands, the subsystems 502-510 may be operated discretely. For example a recognized voice command of "Eject arm 1" may cause the control system 512 to initiate the ejection of the instrument from a teleoperational manipulator at subsystem 502, the subsystem identified as including "arm 1"). Based on the recognized voice commands, the one or all of the subsystems 502-510 may be operated in combination. For example a recognized voice command of "Optimize positioning" may cause the control system 512 to simultaneously or sequentially move the arms of teleoperational manipulators of subsystems 502, 504, 506 to positions and orientations determined to be optimal for the present teleoperational procedure.

The recognized voice communication may be assessed in the context of trigger words alone or together with surgical environment state variables. For example, trigger words "arm 1," "arm 2," and "arm 3" may be associated with subsystems 502, 504, 506, respectively so that voice commands that include those words will be evaluated in the context of the identified subsystem and responses will be implemented in the context of the identified subsystem. Alternatively, the recognized voice communication may be assessed fully in the context of surgical environment state variables, including those associated with subsystems 502-510. example, system monitoring of the surgical state variables allows the recognized voice communication to be assessed in the context of the surgical state variables. If the operator commands, "Swap needle driver," the monitored system state variables will indicate which subsystem 502-510 is operating a needle driver so that the response to the command is implemented on the that subsystem. If the operator commands, "optimize arm positions," the monitored system state variables, providing position and orientation information about each other subsystems, will generate a response that may command a plurality of the subsystems to adjust.

One or more elements in embodiments of the invention may be implemented in software to execute on a processor of a computer system such as control processing system. When implemented in software, the elements of the embodiments of the invention are essentially the code segments to perform the necessary tasks. The program or code segments can be stored in a processor readable storage medium or device that may have been downloaded by way of a computer data signal embodied in a carrier wave over a transmission medium or a communication link. The processor readable storage device may include any medium that can store information including an optical medium, semiconductor medium, and magnetic medium. Processor readable storage device examples include an electronic circuit; a semiconductor device, a semiconductor memory device, a read only memory (ROM), a flash memory, an erasable programmable read only memory (EPROM); a floppy diskette, a CD-ROM, an optical disk, a hard disk, or other storage device, The code segments may be downloaded via computer networks such as the Internet, Intranet, etc.

Note that the processes and displays presented may not inherently be related to any particular computer or other apparatus. Various general-purpose systems may be used with programs in accordance with the teachings herein, or it may prove convenient to construct a more specialized apparatus to perform the operations described. The required structure for a variety of these systems will appear as elements in the claims. In addition, the embodiments of the invention are not described with reference to any particular programming language. It will be appreciated that a variety of programming languages may be used to implement the teachings of the invention as described herein.

While certain exemplary embodiments of the invention have been described and shown in the accompanying drawings, it is to be understood that such embodiments are merely illustrative of and not restrictive on the broad invention, and that the embodiments of the invention not be limited to the specific constructions and arrangements shown and described, since various other modifications may occur to those ordinarily skilled in the art.

## Claims

1. A teleoperational surgical system (10) comprising:
an operator input system (16);
a teleoperational manipulator (12) configured for operation by the operator input system (16), wherein the teleoperational manipulator (12) is coupled to a medical instrument (14) in a surgical environment,
wherein the operator input system (16), the teleoperational manipulator (12), and the medical instrument (14) are a plurality of components of the teleoperational surgical system (10); and
a processing unit (20) including one or more processors (22), wherein the processing unit (20) is configured to
recognize (102) a voice communication;
assess (104) a plurality of surgical environment state variables (200) providing information about a current status of at least one of the plurality of components of the teleoperational surgical system (10),
evaluate (106) the voice communication in a context of the plurality of surgical environment state variables (200) providing information about the current status of at least one of the plurality of components of the teleoperational surgical system (10) by:
referencing a word recognition search space; and
based on the plurality of surgical environment state variables (200), constraining the word recognition search space;
determine (108) a response to the voice communication based on at least one of the plurality of surgical environment state variables (200); and
provide (110) a command to the at least one of the plurality of components of the teleoperational surgical system (10) to implement the response.

2. The system of claim 1 wherein the plurality of surgical environment state variables (200) includes an instrument state variable (206) for the medical instrument (14).

3. The system of claim 1 wherein the plurality of surgical environment state variables (200) includes a manipulator state variable (208) for the teleoperational manipulator (12).

4. The system of claim 1 wherein the plurality of surgical environment state variables (200) includes an operator console variable (210).

5. The system of claim 1 wherein the plurality of surgical environment state variables (200) includes an auxiliary equipment variable (212).

6. The system of claim 1 wherein the plurality of surgical environment state variables (200) includes a patient state variable (216).

7. The system of claim 1 wherein the plurality of surgical environment state variables (200) includes a surgical staff state variable (218).

8. The system of claim 1 wherein the plurality of surgical environment state variables (200) includes a procedure state variable (204).

9. The system of claim 1 wherein the plurality of surgical environment state variables (200) includes a speaker state variable (202).

10. The system of claim 1 wherein providing (110) a command includes displaying a textual message to a user with at least one proposed course of action based on at least one surgical environment state variable (200).

11. The system of claim 1 wherein providing (110) a command includes changing a location of a visualization device in the surgical environment.

12. The system of claim 1 wherein evaluating (106) the voice communication in the context of a plurality of surgical environment state variables (200) includes evaluating a part of speech of the voice communication based on an operator console state variable (210), wherein the operator console state variable (210) is an operator input at an operator input device of the operator input system (16).

13. The system of claim 1 wherein the plurality of surgical environment state variables (200) includes an identified subsystem variable and wherein providing a command to implement the response includes providing a command to implement the response relative to a subsystem associated with the identified subsystem variable.

14. The system of claim 1 wherein evaluating (106) the voice communication includes determining that a voice communication is issued by an operator at the operator input system and providing a command to implement the response includes suppressing an intercom system.

15. The system of claim 1 wherein constraining the word recognition search space includes modifying the word recognition search space to reduce one or more response options from consideration by eliminating at least one word of the word recognition search space based on the plurality of surgical environment state variables (200) or prioritizing at least one word in the word recognition search space based on the plurality of surgical environment state variables (200).

## Patentansprüche

1. Teleoperatives chirurgisches System (10), das Folgendes umfasst:
ein Bedienereingabesystem (16);
einen teleoperativen Manipulator (12), konfiguriert für den Betrieb durch das Bedienereingabesystem (16), wobei der teleoperative Manipulator (12) mit einem medizinischen Instrument (14) in einer chirurgischen Umgebung gekoppelt ist,
wobei das Bedienereingabesystem (16), der teleoperative Manipulator (12) und das medizinische Instrument (14) eine Mehrzahl von Komponenten des teleoperativen chirurgischen Systems (10) sind; und
eine Verarbeitungseinheit (20) mit einem oder mehreren Prozessoren (22), wobei die Verarbeitungseinheit (20) konfiguriert ist zum:
Erkennen (102) einer Sprachkommunikation,
Beurteilen (104) einer Mehrzahl von Chirurgische-Umgebung-Zustandsvariablen (200), die Informationen über einen aktuellen Status von mindestens einer aus der Mehrzahl von Komponenten des teleoperativen chirurgischen Systems (10) bereitstellen,
Auswerten (106) der Sprachkommunikation in einem Kontext der Mehrzahl von Chirurgische-Umgebung-Zustandsvariablen (200), die Informationen über den aktuellen Status von mindestens einer aus der Mehrzahl von Komponenten des teleoperativen chirurgischen Systems (10) bereitstellen, durch:
Referenzieren eines Worterkennungssuchraums; und
Einschränken des Worterkennungssuchraums auf der Basis der Mehrzahl von Chirurgische-Umgebung-Zustandsvariablen (200);
Bestimmen (108) einer Antwort auf die Sprachkommunikation auf der Basis von mindestens einer aus der Mehrzahl von Chirurgische-Umgebung-Zustandsvariablen (200); und
Geben (110) eines Befehls an die mindestens eine aus der Mehrzahl von Komponenten des teleoperativen chirurgischen Systems (10) zum Implementieren der Antwort.

2. System nach Anspruch 1, wobei die Mehrzahl von Chirurgische-Umgebung-Zustandsvariablen (200) eine Instrumentenzustandsvariable (206) für das medizinische Instrument (14) umfasst.

3. System nach Anspruch 1, wobei die Mehrzahl von Chirurgische-Umgebung-Zustandsvariablen (200) eine Manipulatorzustandsvariable (208) für den teleoperierten Manipulator (12) umfasst.

4. System nach Anspruch 1, wobei die Mehrzahl von Chirurgische-Umgebung-Zustandsvariablen (200) eine Bedienkonsolenvariable (210) umfasst.

5. System nach Anspruch 1, wobei die Mehrzahl von Chirurgische-Umgebung-Zustandsvariablen (200) eine Hilfsausrüstungsvariable (212) umfasst.

6. System nach Anspruch 1, wobei die Mehrzahl von Chirurgische-Umgebung-Zustandsvariablen (200) eine Patientenzustandsvariable (216) umfasst.

7. System nach Anspruch 1, wobei die Mehrzahl von Chirurgische-Umgebung-Zustandsvariablen (200) eine Operationspersonal-Zustandsvariable (218) umfasst.

8. System nach Anspruch 1, wobei die Mehrzahl von Chirurgische-Umgebung-Zustandsvariablen (200) eine Prozedurzustandsvariable (204) umfasst.

9. System nach Anspruch 1, wobei die Mehrzahl von Chirurgische-Umgebung-Zustandsvariablen (200) eine Sprecherzustandsvariable (202) umfasst.

10. System nach Anspruch 1, wobei das Geben (110) eines Befehls das Anzeigen einer Textnachricht für einen Benutzer mit mindestens einem vorgeschlagenen Handlungsablauf auf der Basis mindestens einer Chirurgische-Umgebung-Zustandsvariablen (200) umfasst.

11. System nach Anspruch 1, wobei das Geben (110) eines Befehls das Ändern eines Orts eines Visualisierungsgeräts in der chirurgischen Umgebung umfasst.

12. System nach Anspruch 1, wobei das Auswerten (106) der Sprachkommunikation im Kontext einer Mehrzahl von Chirurgische-Umgebung-Zustandsvariablen (200) das Auswerten eines Teils von Sprache der Sprachkommunikation auf der Basis einer Bedienkonsolenzustandsvariablen (210) umfasst, wobei die Bedienkonsolenzustandsvariable (210) eine Bedienereingabe an einem Bedienereingabegerät des Bedienereingabesystems (16) ist.

13. System nach Anspruch 1, wobei die Mehrzahl von Chirurgische-Umgebung-Zustandsvariablen (200) eine identifizierte Subsystemvariable umfasst, und wobei das Geben eines Befehls zum Implementieren der Antwort das Geben eines Befehls zum Implementieren der Antwort relativ zu einem mit der identifizierten Subsystemvariable assoziierten Subsystem umfasst.

14. System nach Anspruch 1, wobei das Auswerten (106) der Sprachkommunikation das Feststellen beinhaltet, dass eine Sprachkommunikation von einem Bediener an dem Bedienereingabesystem ausgegeben wird, und das Geben eines Befehls zum Implementieren der Antwort das Unterdrücken eines Gegensprechsystems umfasst.

15. System nach Anspruch 1, wobei das Einschränken des Worterkennungssuchraums das Modifizieren des Worterkennungssuchraums umfasst, um eine oder mehrere Antwortoptionen durch Eliminieren mindestens eines Worts des Worterkennungssuchraums auf der Basis der Mehrzahl von Chirurgische-Umgebung-Zustandsvariablen (200) oder Priorisieren mindestens eines Worts im Worterkennungssuchraum auf der Basis der mehreren Chirurgische-Umgebung-Zustandsvariablen (200) von der Berücksichtigung auszuschließen.

## Revendications

1. Système chirurgical télécommandé (10) comprenant :
un système de saisie opérateur (16) ;
un manipulateur télécommandé (12) configuré pour être actionné par le système de saisie opérateur (16), le manipulateur télécommandé (12) étant couplé à un instrument médical (14) dans un environnement chirurgical,
dans lequel le système de saisie opérateur (16), le manipulateur télécommandé (12) et l'instrument médical (14) constituent une pluralité de composants du système chirurgical télécommandé (10) ; et
une unité de traitement (20) comprenant un ou plusieurs processeurs (22), l'unité de traitement (20) étant configurée pour
reconnaître (102) une communication vocale ;
évaluer (104) une pluralité de variables d'état d'environnement chirurgical (200) fournissant des informations sur un état courant d'au moins l'un de la pluralité de composants du système chirurgical télécommandé (10),
évaluer (106) la communication vocale dans un contexte de la pluralité de variables d'état d'environnement chirurgical (200) fournissant des informations sur l'état courant d'au moins l'un de la pluralité de composants du système chirurgical télécommandé (10) par :
référencement d'un espace de recherche de reconnaissance de mots ; et
sur la base de la pluralité de variables d'état d'environnement chirurgical (200), limitation de l'espace de recherche de reconnaissance de mots ;
déterminer (108) une réponse à la communication vocale sur la base d'au moins l'une de la pluralité de variables d'état d'environnement chirurgical (200) ; et
présenter (110) une commande à l'au moins l'un de la pluralité de composants du système chirurgical télécommandé (10) pour mettre en œuvre la réponse.

2. Système selon la revendication 1, dans lequel la pluralité de variables d'état d'environnement chirurgical (200) comprend une variable d'état d'instrument (206) pour l'instrument médical (14).

3. Système selon la revendication 1, dans lequel la pluralité de variables d'état d'environnement chirurgical (200) comprend une variable d'état de manipulateur (208) pour le manipulateur télécommandé (12).

4. Système selon la revendication 1, dans lequel la pluralité de variables d'état d'environnement chirurgical (200) comprend une variable de console opérateur (210).

5. Système selon la revendication 1, dans lequel la pluralité de variables d'état d'environnement chirurgical (200) comprend une variable d'équipement auxiliaire (212).

6. Système selon la revendication 1, dans lequel la pluralité de variables d'état d'environnement chirurgical (200) comprend une variable d'état du patient (216).

7. Système selon la revendication 1, dans lequel la pluralité de variables d'état d'environnement chirurgical (200) comprend une variable d'état du personnel chirurgical (218).

8. Système selon la revendication 1, dans lequel la pluralité de variables d'état d'environnement chirurgical (200) comprend une variable d'état de procédure (204).

9. Système selon la revendication 1, dans lequel la pluralité de variables d'état d'environnement chirurgical (200) comprend une variable d'état de haut-parleur (202).

10. Système selon la revendication 1, dans lequel la présentation (110) d'une commande comprend l'affichage d'un message textuel à l'intention d'un utilisateur comportant au moins un plan d'action proposé basé sur au moins une variable d'état d'environnement chirurgical (200).

11. Système selon la revendication 1, dans lequel la présentation (110) d'une commande comprend le changement d'un emplacement d'un dispositif de visualisation dans l'environnement chirurgical.

12. Système selon la revendication 1, dans lequel l'évaluation (106) de la communication vocale dans le contexte d'une pluralité de variables d'état d'environnement chirurgical (200) comprend l'évaluation d'une partie de la parole de la communication vocale sur la base d'une variable d'état de console opérateur (210), la variable d'état de console opérateur (210) étant une saisie de l'opérateur au niveau d'un dispositif de saisie opérateur du système de saisie opérateur (16).

13. Système selon la revendication 1, dans lequel la pluralité de variables d'état d'environnement chirurgical (200) comprend une variable de sous-système identifié, et dans lequel la présentation d'une commande pour mettre en œuvre la réponse comprend la présentation d'une commande pour mettre en œuvre la réponse relativement à un sous-système associé à la variable de sous-système identifié.

14. Système selon la revendication 1, dans lequel l'évaluation (106) de la communication vocale comprend la détermination qu'une communication vocale est émise par un opérateur au niveau du système de saisie opérateur et la présentation d'une commande pour mettre en œuvre la réponse comprend la suppression d'un système d'interphone.

15. Système selon la revendication 1, dans lequel la limitation de l'espace de recherche de reconnaissance de mots comprend une modification de l'espace de recherche de reconnaissance de mots pour réduire une ou plusieurs options de réponse à prendre en compte en éliminant au moins un mot de l'espace de recherche de reconnaissance de mots sur la base de la pluralité de variables d'état d'environnement chirurgical (200) ou en priorisant au moins un mot dans l'espace de recherche de reconnaissance de mots sur la base de la pluralité de variables d'état d'environnement chirurgical (200).
